# DEMANDE DE BREVET EUROPEEN

(11) **EP 1 602 353 A1**
(43) Date de publication de la demande: **07.12.2005**
(21) Numéro de dépôt: 05300399.2
(22) Date de dépôt: 24.05.2005
(51) Int. Cl.: A61K 7/025, A61K 7/027

(54) **Composition cosmétique de maquillage et/ou de soins des lèvres**

(30) Priorité: 28.05.2004 FR 0451055
(71) Demandeur: L'OREAL, 75008 Paris (FR)
(72) Inventeur: Filippi, Vanina, 75015, Paris (FR); Le Chaux, Laure, 94550, Chevilly-Larue (FR)
(74) Mandataire: Le Coupanec, Pascale A.M.P.

(57) **Abrégé**

La présente invention concerne un produit cosmétique pour le soin et/ou le maquillage des lèvres, contenant à titre de charge, des particules de polyuréthane et moins de 15 % en poids d'eau et/ou de solvant hydrosoluble.

## Description

La présente invention se rapporte à une composition cosmétique de maquillage et/ou de soin des lèvres.

Dans le domaine cosmétique, la mise au point de formulations dotées à la fois de bonnes propriétés en terme d'application et de confort, et de propriétés satisfaisantes en terme de tenue, notamment de tenue de la brillance et de non migration, relève d'un objectif permanent.

Les composés classiquement utilisés dans ce type de compositions pour procurer de la brillance sont des huiles visqueuses à fort indice de réfraction, comme le polybutène ou le malate de di-isostéaryle, et/ou des composés pâteux comme le polyacyladipate de diglycéryle.

Cependant, ces composés peuvent dans certaines conditions poser des problèmes de collant et de migration. Par ailleurs, on sait qu'il est possible de limiter la migration des compositions grâce à l'addition de charges. Toutefois, on a aussi constaté que cette addition de charge s'effectuait généralement au détriment de la brillance des compositions obtenues.

En conséquence, il demeure à ce jour un besoin d'une composition de maquillage et/ou de soin des lèvres, acceptable d'un point de vue sensoriel et qui présente une brillance satisfaisante tout en ne migrant sensiblement pas.

De manière inattendue, les inventeurs ont constaté que l'utilisation d'un type de charge particulier, à savoir des particules de polyuréthane, permettait avantageusement d'obtenir des compositions donnant satisfaction en ces termes.

Les particules de polyuréthane de type charges ont été décrites d'une manière générale comme étant utiles pour préparer des compositions cosmétiques dotées notamment de propriétés sensorielles améliorées en terme de douceur. Ainsi par exemple, le document WO 00/56272 prévoit leur utilisation dans une composition de poudre liquide aqueuse qui présente ainsi une grande douceur.

On a maintenant constaté que l'utilisation de particules de polyuréthane de type charge, dans des produits destinés à être appliqués sur les lèvres permettait efficacement de prévenir la migration de la composition appliquée et était particulièrement avantageuse notamment en terme de brillance et de tenue de la brillance de la composition obtenue.

Ces compositions sont en outre très satisfaisantes en terme de facilité d'application, et de confort.

Selon un premier aspect, la présente invention concerne un produit cosmétique pour le soin et/ou le maquillage des lèvres contenant, à titre de charges, des particules de polyuréthane et moins de 15 % en poids d'eau et/ou de solvant hydrosoluble.

Selon un second aspect, la présente invention concerne un produit cosmétique pour le soin et/ou le maquillage des lèvres contenant, à titre de charges, des particules de polyuréthane en une quantité efficace pour ajuster la brillance moyenne à une valeur supérieure ou égale à 30.

Selon un troisième aspect, la présente invention concerne l'utilisation de particules de polyuréthane, notamment conformes à l'invention, à titre de charge dans un produit cosmétique de maquillage et/ou de soin des lèvres ayant une brillance moyenne supérieure ou égale à 30.

Selon un quatrième aspect, la présente invention concerne un procédé de maquillage dans lequel on applique sur les lèvres, un produit tel que défini précédemment.

Au sens de la présente invention, on entend désigner par le qualificatif de « charges » des particules de toutes formes qui sont insolubles dans le milieu de la composition, quelle que soit la température à laquelle la composition est fabriquée, et qui demeurent sous forme de particules y compris après application de la composition. En particulier, les charges selon l'invention ne sont pas filmogènes : elles ne sont pas aptes à former un film continu et adhérent sur un support, notamment sur les lèvres, et en particulier un film cohésif et mieux encore, un film dont la cohésion et les propriétés mécaniques sont telles que ledit film peut être isolé dudit support.

Par « brillance moyenne », on désigne la brillance telle qu'elle peut être mesurée à l'aide d'un brillancemètre, de manière conventionnelle par la méthode suivante.

Sur une carte de contraste de marque LENETA et de référence FORM 1A PENOPAC, on étale une couche comprise entre 50 µm et 150 µm d'épaisseur d'une composition à l'aide d'un étaleur automatique.

La couche de composition recouvre au moins le fond blanc de la carte. On laisse sécher le dépôt 24 heures à une température de 30 °C, puis on procède à la mesure de la brillance à 20° sur le fond blanc à l'aide d'un brillancemètre de marque BYK GARDNER et de référence microTRI-GLOSS.

Cette mesure (comprise entre 0 et 100) est répétée au moins trois fois, et la brillance moyenne est la moyenne d'au moins trois mesures effectuées.

Avantageusement, la composition conforme à l'invention peut présenter une brillance moyenne supérieure ou égale à une valeur de 30, de préférence 40, de préférence encore 50, de préférence encore 60, en particulier supérieure ou égale à 65, et notamment supérieure ou égale à 70.

Par l'expression « particules de polyuréthane », on entend désigner dans la présente invention des particules constituées d'un matériau au moins en partie de type polyuréthane.

Elles sont avantageusement sous une forme réticulée.

Les particules utilisées dans la présente invention ont, selon un mode de réalisation particulier, une taille moyenne allant de 4 à 20 µm, et en particulier de 5 à 15 µm.

Les particules utilisées dans la présente invention sont généralement sensiblement sphériques.

Selon un mode de réalisation particulier, les particules de polyuréthane utilisées sont des particules comprenant un copolymère, ledit copolymère comprenant du triméthylol hexyllactone. En particulier, il peut s'agir d'un polymère d'hexaméthylène di-isocyanate/triméthylol hexyllactone. De telles particules sont notamment disponibles dans le commerce, par exemple sous la dénomination de PLASTIC POWDER D-400® ou PLASTIC POWDER D-800® de la société TOSHIKI.

Les particules de polyuréthane sont utilisées dans les compositions de l'invention en une quantité telle que la brillance moyenne de la composition est supérieure ou égale à 30, de préférence 40, de préférence encore 50, de préférence encore 60, en particulier supérieure ou égale à 65, et notamment supérieure ou égale à 70.

En particulier, ces particules peuvent être présentes en une teneur allant de 0,1 % à 99 %, en particulier de 0,1 % à 30 %, plus particulièrement de 0,1 à 15 %, voire de 0,1 % à 7 % en poids par rapport au poids total du produit conforme à l'invention.

Le produit cosmétique de l'invention comporte généralement au moins une phase grasse et notamment au moins un corps gras liquide à température ambiante (25 °C) et à pression atmosphérique et/ou un corps gras solide à température ambiante et à pression atmosphérique. La phase grasse peut en outre contenir des agents gélifiants et structurants d'huiles de nature organique et/ou des solvants organiques lipophiles.

La phase grasse du produit selon l'invention peut notamment comprendre, à titre de corps gras liquide, au moins une huile volatile ou non volatile ou un de leurs mélanges.

Par « huile volatile », on entend au sens de l'invention toute huile susceptible de s'évaporer au contact de la peau ou des lèvres en moins d'une heure, à température ambiante et pression atmosphérique. Les huiles volatiles de l'invention sont des huiles cosmétiques volatiles, liquides à température ambiante, ayant une pression de vapeur non nulle, à température ambiante et pression atmosphérique, allant en particulier de 0,01 à 300 mm de Hg (1,33 Pa à 40.000 Pa) et de préférence supérieure à 0,3 mm de Hg (30 Pa).

Par « huile non volatile », on entend une huile restant sur la peau ou les lèvres à température ambiante et pression atmosphérique au moins plusieurs heures et ayant notamment une pression de vapeur inférieure à 0,01 mm de Hg (1,33 Pa).

Ces huiles volatiles ou non volatiles peuvent être des huiles hydrocarbonées notamment d'origine animales ou végétales, des huiles siliconées, ou leurs mélanges. On entend par « huile hydrocarbonée », une huile contenant principalement des atomes d'hydrogène et de carbone et éventuellement des atomes d'oxygène, d'azote, de soufre et/ou de phosphore.

Comme précisé précédemment, ces huiles peuvent être plus particulièrement choisies de manière à ajuster, conjointement avec les charges selon l'invention, le degré de brillance souhaité à ce produit cosmétique.

Les huiles hydrocarbonées volatiles peuvent être choisies parmi les huiles hydrocarbonées ayant de 8 à 16 atomes de carbone, et notamment les alcanes ramifiés en C₈-C₁₆ comme les isoalcanes en C₈-C₁₆ d'origine pétrolière (appelées aussi isoparaffines) comme l'isododécane (encore appelé 2,2,4,4,6-pentaméthylheptane), l'isodécane, l'isohexadécane, et par exemple les huiles vendues sous les noms commerciaux d'Isopars® ou de Permethyls®, les esters ramifiés en C₈-C₁₆ tels que le néopentanoate d'iso-hexyle, et leurs mélanges. D'autres huiles hydrocarbonées volatiles comme les distillats de pétrole, notamment ceux vendus sous la dénomination Shell Solt® par la société SHELL, peuvent aussi être utilisées.

Comme huiles volatiles, on peut aussi utiliser les silicones volatiles, comme par exemple les huiles de silicones linéaires ou cycliques volatiles, notamment celles ayant une viscosité ≤ 8 centistokes (8 x 10⁻⁶ m²/s), et ayant notamment de 2 à 7 atomes de silicium, ces silicones comportant éventuellement des groupes alkyle ou alcoxy ayant de 1 à 10 atomes de carbone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthyl hexyltrisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

L'huile volatile peut être présente dans le produit selon l'invention à une teneur allant de 0,1 % à 98 % en poids, notamment de 1 % à 65 % en poids, et en particulier de 2 % à 50 % en poids, par rapport au poids total du produit.

Les huiles non volatiles peuvent notamment être choisies parmi les huiles hydrocarbonées le cas échéant fluorées et/ou les huiles siliconées non volatiles.

Comme huile hydrocarbonée non volatile, on peut notamment citer :
- les huiles hydrocarbonées d'origine animale,
- les huiles hydrocarbonées d'origine végétale telles que les triglycérides constitués d'esters d'acides gras et de glycérol dont les acides gras peuvent avoir des longueurs de chaînes variées de C₄ à C₂₄, ces dernières pouvant être linéaires ou ramifiées, saturées ou insaturées ; ces huiles sont notamment les huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de courge, de cassis, d'onagre, de millet, d'orge, de quinoa, de seigle, de carthame, de bancoulier, de passiflore, de rosier muscat ; le beurre de karité ; ou encore les triglycérides des acides caprylique/caprique comme ceux vendus par la société Stéarineries Dubois ou ceux vendus sous les dénominations Miglyol 810®, 812® et 818® par la société Dynamit Nobel,
- les éthers de synthèse ayant de 10 à 40 atomes de carbone ;
- les hydrocarbures linéaires ou ramifiés, d'origine minérale ou synthétique tels que la vaseline, les polydécènes, le polyisobutène hydrogéné tel que le parléam, le squalane et leurs mélanges,
- les esters de synthèse comme les huiles de formule R₁COOR₂ dans laquelle R₁ représente le reste d'un acide gras linéaire ou ramifié comportant de 1 à 40 atomes de carbone et R₂ représente une chaîne hydrocarbonée notamment ramifiée contenant de 1 à 40 atomes de carbone à condition que R₁ + R₂ soit ≥ 10, comme par exemple l'huile de Purcellin (octanoate de cétostéaryle), le myristate d'isopropyle, le palmitate d'isopropyle, les benzoates d'alcools en C₁₂ à C₁₅, le laurate d'hexyle, l'adipate de diisopropyle, l'isononanoate d'isononyle, le palmitate de 2-éthyl-hexyle, l'isostéarate d'isostéaryle, des heptanoates, octanoates, décanoates ou ricinoléates d'alcools ou de polyalcools comme le dioctanoate de propylène glycol ; les esters hydroxylés comme le lactate d'isostéaryle, le malate de di-isostéaryle ; les esters de polyols et les esters du pentaérythritol,
- les alcools gras liquides à température ambiante à chaîne carbonée ramifiée et/ou insaturée ayant de 12 à 26 atomes de carbone comme l'octyl dodécanol, l'alcool isostéarylique, l'alcool oléique, le 2-hexyldécanol, le 2-butyloctanol, et le 2-undécylpentadécanol,
- les acides gras supérieurs tels que l'acide oléique, l'acide linoléique, l'acide linolénique et leurs mélanges.

Les huiles de silicone non volatiles utilisables dans le produit conforme à l'invention peuvent être les polydiméthylsiloxanes (PDMS) non volatiles, les polydiméthylsiloxanes comportant des groupements alkyle ou alcoxy pendants et/ou en bouts de chaîne siliconée, groupements ayant chacun de 2 à 24 atomes de carbone, les silicones phénylées comme les phényl triméthicones, les phényl diméthicones, les phényl triméthylsiloxy diphénylsiloxanes, les diphényl diméthicones, les diphényl méthyldiphényl trisiloxanes, et les 2-phényléthyl triméthylsiloxysilicates et leurs mélanges.

Les huiles non volatiles peuvent être présentes dans le produit selon l'invention en une teneur allant de 0,01 à 90 % en poids, notamment de 0,1 % à 85 % en poids, et en particulier de 1 % à 70 % en poids par rapport au poids total du produit.

Les huiles peuvent représenter de 0,01 % à 99 % du poids total du produit, en particulier de 0,05 % à 60 % et plus particulièrement de 1 % à 60 % en poids par rapport au poids total du produit.

Dans le cadre de la présente invention, on utilise avantageusement au moins une huile dont la masse molaire est comprise entre 650 et 10.000 g/mol, et en particulier entre 750 et 7.500 g/mol. Selon un mode de mise en oeuvre, le produit de l'invention comprend une phase huileuse comprenant au moins 70 % en poids d'une huile de masse molaire comprise entre 650 et 10.000 g/mol, en particulier entre 750 et 7.500 g/mol. La phase huileuse comprend avantageusement plus de 80 %, en particulier plus de 85 % en poids d'un huile de masse molaire comprise entre 650 et 10.000 g/mol, et en particulier entre 750 et 7.500 g/mol. L'huile de masse molaire élevée est en particulier choisie parmi:
- les esters d'acides gras linéaires ayant un nombre total de carbone allant de 35 à 70,
- les esters hydroxylés,
- les esters aromatiques,
- les esters d'alcool gras ou d'acides gras ramifiés en C₂₄-C₂₈,
- les huiles siliconées,
- les huiles d'origine végétale,
et leurs mélanges.

L'huile de masse molaire élevée est en particulier choisie parmi les polybutylènes, les polyisobutylènes hydrogénés, les polydécènes, les polydécènes hydrogénés, les copolymères de la vinylpyrrolidone tel que le copolymère PVP / hexadécène, le tétrapélargonate de pentaérythrityle, le triisostéarate de polyglycérol-2, le trimellitate de tridécyle, le citrate de triisoarachidyle, le tétraisononanoate de pentaérythrityle, le triisostéarate de glycéryle, le tétraisostéarate de pentaérythrityle, le tri décyl-2 tétradécanoate de glycéryle, les silicones phénylées, l'huile de sésame, et leurs mélanges.

Les huiles de masse molaire élevée citées précédemment sont généralement des huiles brillantes.

Dans un mode de réalisation particulier de l'invention, on utilise au moins une huile de masse molaire élevée en association avec une poudre de polyuréthane.

Les produits cosmétiques obtenus sont particulièrement satisfaisants en terme de brillance.

Avantageusement, le rapport entre la teneur en poids de l'huile de masse molaire élevée et la teneur en poids de particules de polyuréthane telles que définies précédemment est de 1 à 30, en particulier de 1 à 20, et plus particulièrement de 1 à 15.

Selon une variante particulière, le produit cosmétique conforme à l'invention peut comporter une phase huileuse ayant un indice de réfraction compris entre 1,46 et 1,51, ce qui peut permettre d'obtenir une brillance relativement élevée.

Plus généralement, le corps gras liquide à température ambiante et à pression atmosphérique peut être présent à raison de 0,01 à 99 % en poids et notamment de 0,1 à 85 % en poids, par rapport au poids de la phase grasse.

La composition peut comprendre au moins une cire de 0,01 à 70 %, notamment de 0,1 à 65 % et en particulier de 1 à 65 % en poids, par rapport au poids total de la phase grasse.

Par « cire » au sens de la présente invention, on entend un composé gras lipophile, solide à température ambiante (25 °C), à changement d'état solide/liquide réversible, ayant une température de fusion supérieure à 30 °C pouvant aller jusqu'à 200 °C, une dureté supérieure à 0,5 MPa, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les cires utilisables dans l'invention sont des composés solides à température ambiante, destinés à structurer la composition en particulier sous forme de stick ; elles peuvent être hydrocarbonées, fluorées et/ou siliconées et être d'origine végétale, minérale, animale et/ou synthétique. En particulier, elles présentent une température de fusion supérieure à 40 °C et mieux supérieure à 45 °C.

Comme cire utilisable dans l'invention, on peut citer celles généralement utilisées dans le domaine cosmétique : elles sont notamment d'origine naturelle comme la cire d'abeilles, la cire de Carnauba, de Candelilla, d'Ouricoury, du Japon, de fibres de liège ou de canne à sucre, de riz, de Montan, la paraffine, les cires de lignite ou microcristalline, la cérésine ou l'ozokérite, les huiles hydrogénées comme l'huile de jojoba ; les cires synthétiques comme les cires de polyéthylène issues de la polymérisation ou copolymérisation de l'éthylène et les cires de Fischer-Tropsch ou encore des esters d'acides gras comme l'octacosanyl stéarate, les glycérides concrets à 40 °C et mieux à 45 °C, les cires de silicones comme les alkyl- ou alkoxydiméthicones ayant une chaîne alkyle ou alcoxy de 10 à 45 atomes de carbone, les esters de poly(di)méthylsiloxane solide à 40 °C dont la chaîne ester comporte au moins 10 atomes de carbone ; et leurs mélanges.

La quantité en cire est notamment déterminante pour conférer un niveau de dureté donné. Cette dureté peut être mesurée par la méthode dite « du fil à couper le beurre », qui consiste à couper un bâton de rouge à lèvres de 12,7 mm et à mesurer la dureté à 20 °C, au moyen d'un dynamomètre DFGHS 2® de la société Indelco-Chatillon se déplaçant à une vitesse de 100 mm/minute. Elle est exprimée comme la force de cisaillement (exprimée en gramme) nécessaire pour couper un bâton dans ces conditions. Selon cette méthode la dureté d'un produit conforme à l'invention en bâton de type stick, c'est-à-dire en cylindre ayant un diamètre d'environ 12,7 mm, va notamment de 50 à 300 g, en particulier de 100 à 250 g et par exemple de 100 à 230 g. Lorsque le produit est formulé en bâton de type crayon à lèvres, c'est-à-dire en cylindre ayant un diamètre d'environ 8,3 mm, sa dureté est généralement comprise entre 30 et 250, en particulier entre 50 et 240, et plus particulièrement entre 50 et 150 g.

Les produits conformes à l'invention comprennent généralement moins de 15 % d'eau et/ou de solvant hydrosoluble.

Par « solvant hydrosoluble », on désigne un composé liquide à température ambiante et miscible à l'eau (miscibilité dans l'eau supérieure à 50 % en poids à 25 °C et pression atmosphérique).

Les solvants hydrosolubles considérés sont généralement en outre volatils. Parmi ces solvants hydrosolubles, on peut citer notamment les mono-alcools inférieurs ayant de 1 à 5 atomes de carbone tels que l'éthanol et l'isopropanol, les glycols ayant de 2 à 8 atomes de carbone tels que l'éthylène glycol, le propylène glycol, le 1,3 butylène glycol et le di-propylène glycol, les cétones en C₃ et C₄ et les aldéhydes en C₂ à C₄.

Selon un mode de réalisation particulier de l'invention, le produit a une teneur en eau et/ou en solvant hydrosoluble inférieure ou égale à 10 %, et en particulier inférieure ou égale à 5 %.

Selon un mode de réalisation particulier, il s'agit d'un produit sensiblement exempt d'eau et de solvant hydrosoluble, c'est-à-dire d'un produit anhydre.

La composition peut contenir outre la poudre de polyuréthanne décrite précédemment une autre charge. Il peut notamment s'agir de charges comme par exemple le talc, le stéarate de zinc, le mica, le kaolin, les poudres de polyamide (Nylon®) (Orgasol® de chez ATOCHEM), les poudres de polyéthylène, les poudres de polymères de tétrafluoroéthylène (Téflon®), l'amidon, le nitrure de bore, des microsphères polymériques telles que celles de chlorure de polyvinylidène/acrylonitrile comme l'Expancel® (NOBEL INDUSTRIE), de copolymères d'acide acrylique (Polytrap® de la société DOW CORNING), les microbilles de résine de silicone (Tospearls® de TOSHIBA, par exemple), et les organopolysiloxanes élastomères.

Selon une variante particulière de l'invention, les produits cosmétiques comprennent moins de 10 % en poids, en particulier moins de 7 % en poids, notamment moins de 5 % en poids de charge additionnelle par rapport au poids total de la composition.

Le produit de l'invention, comprend selon une variante au moins un agent de coloration pouvant notamment être présent à raison de 0,01 % à 40 % en poids, notamment de 0,01 % à 30 % en poids et en particulier de 0,05 % à 25 % en poids, par rapport au poids total du produit.

Ces ou cet agent de coloration peuvent être choisis parmi les pigments, les colorants hydrosolubles ou liposolubles, les nacres, les paillettes et leurs mélanges.

Par pigments, il faut comprendre des particules blanches ou colorées, minérales ou organiques, insolubles dans la phase hydrophile liquide, destinées à colorer et/ou opacifier la composition. Par nacres, il faut comprendre des particules irisées, notamment produites par certains mollusques dans leur coquille ou bien synthétisées.

On peut citer, parmi les pigments minéraux, le dioxyde de titane, éventuellement traité en surface, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, les poudres métalliques comme la poudre d'aluminium, la poudre de cuivre.

Parmi les pigments organiques, on peut citer le noir de carbone, les pigments de type D & C, et les laques à base de carmin de cochenille, de baryum, strontium, calcium, aluminium.

On peut également citer les pigments à effet tels les particules comportant un substrat organique ou minéral, naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, l'oxyde de chrome et leurs mélanges.

Les pigments nacrés peuvent être choisis parmi le mica recouvert de titane ou d'oxychlorure de bismuth, le mica titane recouvert avec des oxydes de fer, le mica titane recouvert avec notamment du bleu ferrique ou de l'oxyde de chrome, le mica titane recouvert avec un pigment organique du type précité ainsi que les pigments nacrés à base d'oxychlorure de bismuth. On peut également utiliser les pigments interférentiels, notamment à cristaux liquides ou multicouches.

Les colorants hydrosolubles sont par exemple le jus de betterave, le bleu de méthylène.

Le produit selon l'invention peut, de plus, comprendre tous les ingrédients classiquement utilisés dans les domaines concernés et plus spécialement dans le domaine cosmétique et dermatologique. Ces ingrédients sont en particulier choisis parmi les vitamines, les antioxydants, les épaississants, les oligo-éléments, les adoucissants, les séquestrants, les parfums, les agents alcalinisants ou acidifiants, les conservateurs, les filtres UV, les actifs hydrophiles ou lipophiles et leurs mélanges. Les quantités de ces différents ingrédients sont celles classiquement utilisées dans les domaines concernés et par exemple de 0,01 % à 20 % du poids total de la composition.

Bien entendu, l'homme du métier veillera à choisir ce ou ces éventuels composés complémentaires, et/ou leur quantité, de manière telle que les propriétés avantageuses du produit selon l'invention ne soient pas, ou substantiellement pas, altérées par l'adjonction considérée.

Le produit de l'invention peut être obtenu selon les procédés de préparation classiquement utilisés en cosmétique ou en dermatologie.

Le produit de l'invention peut se présenter sous la forme de composition solide, compactée ou coulée en stick ou en coupelle, pâteuse ou liquide.

Avantageusement, il se présente sous forme solide, à savoir sous forme dure (ne s'écoulant pas sous son propre poids) notamment coulée ou compactée, par exemple en stick ou en coupelle.

Selon une variante particulière de l'invention, il se présente sous la forme de rouges à lèvres ou de baumes à lèvres.

Le produit conforme à l'invention peut encore être sous la forme d'un « gloss liquide ». On désigne, par l'expression « gloss liquide », de même que par les expressions « rouge à lèvres liquide » ou « brillant à lèvres », un produit fluide destiné à être appliqué sur les lèvres et conditionné par exemple dans un récipient pourvu d'un applicateur, cet applicateur comportant un organe de préhension qui sert également de capuchon de fermeture du récipient, et un élément d'application.

Un tel produit peut se présenter sous la forme d'une pâte, ou d'une crème. Il peut être une émulsion huile-dans-eau ou eau-dans-huile, un gel anhydre, solide ou souple ou encore sous forme de poudre libre ou compactée et même sous forme biphasique. Selon une variante particulière, il se présente sous la forme d'une émulsion.

Le produit selon l'invention peut se présenter sous la forme d'un produit, coloré ou non, sous forme d'un produit de protection solaire. Il contient notamment des actifs cosmétiques. Il peut alors être utilisé comme base de soin ou de traitement pour les lèvres comme des baumes à lèvres, protégeant les lèvres du froid et/ou du soleil et/ou du vent. Comme actif cosmétique utilisable dans l'invention, on peut citer les vitamines A, E, C, B3, les provitamines comme le D-panthénol, les actifs apaisants comme l'α-bisabolol, l'aloe vera, l'allantoïne, les extraits de plantes ou les huiles essentielles, les agents protecteurs ou restructurants comme les céramides, les actifs fraîcheur comme le menthol et ses dérivés, les émollients (beurre de cacao), les hydratants (arginine PCA), les actifs antirides, les acides gras essentiels, et leurs mélanges.

La composition de l'invention peut également se présenter sous la forme d'un produit de maquillage des lèvres comme un rouge ou un brillant à lèvres, présentant éventuellement des propriétés de soin ou de traitement.

Bien entendu, la composition de l'invention doit être cosmétiquement acceptable, à savoir non toxique et susceptible d'être appliquée sur la peau et les lèvres d'êtres humains.

Les exemples ci-après sont donnés à titre illustratif et sans caractère limitatif

### Exemple 1

Deux formulations de rouges à lèvres sont préparées :
- l'une conforme à l'invention, incorpore comme charge le copolymère réticulé hexaméthylène di-isocyanate/triméthylol hexyllactone commercialisé sous la dénomination Plastic Powder D400® par la société TOSHIKI, et
- l'autre, dite comparative, remplace ladite charge par une quantité équivalente d'un mélange de charges utilisé classiquement dans les compositions de rouges à lèvres (lauroyl lysine / kaolin).

Leurs compositions sont les suivantes :

| Phase | Matières premières | Formulation selon l'invention | Formulation témoin |
|---|---|---|---|
| A | Malate de diisostéaryle | 30,99 | 30,99 |
| | Copolymère de vinylpyrrolidone/hexadécène | 7,74 | 7,74 |
| | Polylaurate de vinyle | 2,62 | 2,62 |
| | Néopentanoate d'isodécyle | 15,89 | 15,89 |
| | Butyl hydroxy toluène | 0,06 | 0,06 |
| A' | Polybutène | 7,00 | 7,00 |
| | Phényl triméthicone (« Belsil PDM 1000» de WACKER) | 7,60 | 7,60 |
| | Phényl triméthicone (« DC 556» de Dow Corning) | 2,80 | 2,80 |
| | Oléate de sorbitan | 7,30 | 7,30 |
| B | Cire de polyéthylène (« Performalène 500 » de New Phase Technologies ») | 6,33 | 6,33 |
| | Cire de polyéthylène (« Performalène 400 » de New Phase Technologies ») | 2,10 | 2,10 |
| | Copolymère stéarate d'allyle / acétate de vinyle (« Méxomère PQ » de CHIMEX») | 0,77 | 0,77 |
| C | Pigments et Nacres | 5,80 | 5,80 |
| D | N-Lauroyl L-lysine | - | 1,24 |
| | Silicate d'aluminium | - | 1,76 |
| | Plastic Powder D400® | 3,00 | - |
| | Total | 100,00 | 100,00 |

### Le protocole de préparation est le suivant :

Les pigments et charges (phases C sans les nacres et D) sont broyés à l'aide d'une broyeuse tricylindres dans la phase huileuse (phase A + A'). On introduit alors le mélange obtenu ainsi que les cires (phase B) dans un poêlon. On chauffe à 98-100 °C tout en homogénéisant le mélange à l'aide d'un barreau aimanté. On ajoute les nacres 5 minutes avant le coulage. La pâte à 98-100 °C est alors coulée dans un moule à 42 °C. Une fois la pâte figée, le moule est placé au congélateur à -18 °C pendant une demi-heure. On procède ensuite au démoulage des sticks.

Ces deux compositions sont testées par trois personnes volontaires.

Il ressort de ces tests que la composition selon l'invention est ressentie comme étant plus onctueuse lors de son application que la formulation témoin. Elle présente une meilleure brillance à l'application, qu'elle conserve aussi mieux que la composition témoin. De plus, la composition selon l'invention migre moins que la formulation témoin.

### Exemple 2

Selon le même protocole que celui décrit à l'exemple 1, on a préparé la composition de rouge à lèvres suivante :

| | |
|---|---|
| Malate de diisostéaryle | 29,37 |
| Copolymère de vinylpyrrolidone/hexadécène | 7,33 |
| Polylaurate de polyvinyle | 2,48 |
| Néopentanoate d'isodécyle | 15,06 |
| Butyl hydroxy toluène | 0,06 |
| Polybutène | 7,00 |
| Phényl triméthicone (« Belsil PDM 1000» de WACKER) | 7,60 |
| Phényl triméthicone (« DC 556» de Dow Corning) | 2,80 |
| Oléate de sorbitan | 7,30 |
| Cire de polyéthylène | |
| (« Performalène 500 » de New Phase Technologies ») | 6,33 |
| Cire de polyéthylène | |
| (« Performalène 400 » de New Phase Technologies ») | 2,10 |
| Copolymère stéarate d'allyle / acétate de vinyle | 0,77 |
| Pigments et nacres | 6,80 |
| Plastic Powder D400® | 5,00 |

A l'application, cette composition est ressentie à la fois comme plus onctueuse et plus brillante. De plus, la brillance et la sensation de confort durent plus longtemps. En outre, cette composition migre moins.

## Revendications

1. Produit cosmétique pour le soin et/ou le maquillage des lèvres, contenant à titre de charge, des particules de polyuréthane et moins de 15 % en poids d'eau et/ou de solvant hydrosoluble.

2. Produit selon la revendication 1, **caractérisé en ce que** sa teneur en eau et/ou en solvant hydrosoluble est inférieure ou égale à 10 %, et en particulier inférieure ou égale à 5 % en poids.

3. Produit cosmétique pour le soin et/ou le maquillage des lèvres, contenant à titre de charge, des particules de polyuréthane en une quantité telle que la brillance moyenne de la composition est supérieure ou égale à 30.

4. Produit selon l'une quelconque des revendications 1 à 3, **caractérisé en ce que** sa brillance moyenne est supérieure ou égale à 40, 50, 60, 65, et en particulier supérieure ou égale à 70.

5. Produit selon la revendication 3 ou 4, **caractérisé en ce qu'**il comprend moins de 15 %, en particulier moins de 10 %, plus particulièrement moins de 5 % en poids d'eau et/ou de solvant hydrosoluble par rapport au poids total dudit produit.

6. Produit selon l'une quelconque des revendications précédentes, **caractérisé en ce que** lesdites particules ont une taille moyenne en allant de 4 à 20 µm, et en particulier de 5 à 15 µm.

7. Produit selon l'une quelconque des revendications précédentes, **caractérisé en ce que** lesdites particules sont sensiblement sphériques.

8. Produit selon l'une quelconque des revendications précédentes, **caractérisé en ce que** ledit polyuréthane est un copolymère de triméthylol hexyllactone.

9. Produit selon la revendication 8, **caractérisé en ce que** ledit copolymère est un copolymère hexaméthylène di-isocyanate/triméthylol hexyllactone.

10. Produit selon l'une quelconque des revendications précédentes, **caractérisé en ce que** lesdites particules sont présentes en une teneur allant de 0,1 % à 99 %, en particulier allant de 0,1 % à 30 %, plus particulièrement de 0,1 % à 15 %, voire de 0,1 % à 7 % en poids par rapport au poids total dudit produit.

11. Produit selon l'une quelconque des revendications précédentes, **caractérisé en ce qu'**il comprend au moins une phase grasse.

12. Produit selon la revendication 11, **caractérisé en ce que** la phase grasse comprend une huile choisie parmi les polybutènes, les polyisobutylènes hydrogénés, les polydécènes, les polydécènes hydrogénés, les copolymères de la vinylpyrrolidone tel que le copolymère polyvinylpyrrolidone / hexadécène, le tétrapélargonate de pentaérythrityle, le triisostéarate de polyglycérol-2, le trimellitate de tridécyle, le citrate de triisoarachidyle, le tétraisononanoate de pentaérythrityle, le triisostéarate de glycéryle, le tétraisostéarate de pentaérythrityle, le tri décyl-2 tétradécanoate de glycéryle, les silicones phénylées, et des huiles végétales telles que l'huile de sésame.

13. Produit selon l'une quelconque des revendications précédentes, **caractérisé en ce qu'**il comprend au moins un agent de coloration à raison de 0,01 à 40 %, notamment de 0,01 à 30 %, et en particulier de 0,05 à 25 %, en poids par rapport au poids total de dudit produit.

14. Produit selon la revendication 13, **caractérisé en ce que** ledit agent de coloration est choisi parmi les pigments, les nacres, les colorants hydrosolubles ou liposolubles et leurs mélanges.

15. Produit selon l'une quelconque des revendications précédentes, **caractérisé en ce qu'**il se présente sous la forme de produits coulés en stick ou en coupelle.

16. Produit selon l'une quelconque des revendications 1 à 14, **caractérisé en ce qu'**il se présente sous la forme d'un gloss liquide.

17. Utilisation à titre de charge de particules de polyuréthane, dans un produit cosmétique pour le maquillage et/ou le soin des lèvres ayant une brillance moyenne supérieure ou égale à 30.

18. Utilisation selon la revendication 17, **caractérisée en ce que** les particules de polyuréthane sont telles que défmies en revendications 3 à 10.

19. Procédé de maquillage des lèvres comprenant au moins l'application sur les lèvres d'un produit selon l'une quelconque des revendications 1 à 16.
